# EUROPÄISCHE PATENTANMELDUNG

(11) **EP 1 184 615 A1**
(43) Veröffentlichungstag der Anmeldung: **06.03.2002**
(21) Anmeldenummer: 01120059.9
(22) Anmeldetag: 21.08.2001
(51) Int. Cl.: F16M 11/12, G02B 7/00, A61B 19/00

(54) **Stativanordnung**

(30) Priorität: 29.08.2000 DE 10042272
(71) Anmelder: Leica Microsystems AG, 9435 Heerbrugg (CH)
(72) Erfinder: Sander, Ulrich, Dr., 9445 Rebstein (CH)
(74) Vertreter: Reichert, Werner F., Dr.

(57) **Zusammenfassung**

Stativanordnung, insbesondere für medizinische Untersuchungs- und Operationsmikroskope, mit einem Ständerfuss (1), einem gegenüber dem Ständerfuss (2) um eine vertikale Achse (A) verdrehbaren Oberteil (2a) und einem daran schwenkbar gelagerten Ausleger (3) zur Aufnahme des Mikroskops (9). Das Lampengehäuse (11) ist an einem mit dem Ausleger (3) verbundenen Träger (10) angeordnet und dient somit dem Ausbalancieren des Stativs.

## Beschreibung

Die Erfindung betrifft eine Stativanordnung, insbesondere für medizinische Untersuchungs- und Operationsmikroskope, gemäss dem Oberbegriff des Anspruches 1.

Stative für Operationsmikroskope müssen einerseits sehr beweglich und gut ausbalancierbar sein, damit das Bewegen des Mikroskops mit geringem Kraftaufwand und in sehr kurzer Zeit erfolgen kann. Andererseits muss das Stativ sehr steif sein, um eine gute Bildqualität zu ermöglichen und ein Verwackeln des Bildes zu verhindern. Wegen der für die Anwendung am Operationstisch erforderlichen, relativ grossen Ausladung des Auslegers entstehen an den Bauteilen und Lagerstellen hohe Kräfte und Biegemomente.

Um die Operationsstelle optimal auszuleuchten ist eine relativ starke Lichtquelle erforderlich. Diese wird in der Regel in ein als separates Bauteil ausgebildetes Lampengehäuse integriert. Damit dieses dem operierenden Arzt beim Operieren nicht im Weg steht und auch damit die vom Lampengehäuse abstrahlende Wärme den Arzt nicht behindert, ist es üblich, das Lampengehäuse weit weg vom Operationsmikroskop, z.B. am Ständerfuss, anzuordnen. Das Licht wird mittels eines flexiblen Lichtleiters zur Operationsstelle geleitet. Diese Anordnung des Lampengehäuses erfordert sehr lange Lichtleiter. Da diese Lichtleiter an den vielen Gelenken des Stativs vorbeigeführt werden müssen, ist eine sehr hohe Flexibilität gefordert.

Weniger Flexibilität fordert ein bekannter Stativ-Aufbau "Zeiss Visu 200 & S8". Dieser Aufbau verfügt auch über einen Scherenarm, der den Ausleger bildet. Der Scherenarm ist durch eine Stützfeder federbelastet und erlaubt somit einen Gewichtsausgleich, so dass ein Anwender ein relativ austariertes Mikroskop bedienen kann. "Relativ austariert" bedeutet in diesem Fall, dass der Anwender das Gewicht des Mikroskops kaum spürt, weil es durch die Kraft der Stützfeder im Rahmen der Scherenarmkonstruktion aufgehoben wird. Diese scheinbare Balancierung erfolgt jedoch nicht über einen Gewichtsausgleich über eine senkrechte Drehachse bzw. Stützachse des Stativs. Es werden vielmehr die Last des Scherenarms und sämtliche Lasten, die an ihm hängen - inklusive z.B. das Gewicht der Stützfeder und das Mikroskop samt Zubehör - in Form von Biegekräften an der senkrechten Stützachse aufgenommen. Diese Biegekräfte führen zu einem Kippmoment, das wiederum durch einen genügend grossen und schweren Stützfuss unterhalb der senkrechten Stützachse aufgenommen wird.

Je größer die Lasten sind, desto grösser werden die Biegekräfte und je größer die Kippmomente sind, umso grösser und schwerer muss der Stützfuss sein. Je grösser jedoch der Stützfuss ist, umso weniger Bewegungsfreiheit ergibt sich für den Anwender bei seiner Tätigkeit im Bereich des Stativs.

Aus der WO 9901693 A1 ist ein Mikroskopstativ bekannt, bei dem dieses Problem dadurch gelöst wird, dass die Montage der Steuerung, der Beleuchtung usw. an der senkrechten Stützachse, in einem vom Mikroskop abragenden Bereich vorgenommen wird.

Einen gewissen Gewichtsausgleichseffekt oder zumindest eine Verlagerung des Gesamtschwerpunktes in Richtung der senkrechten Stützachse mag auch der erwähnte Stativ-Aufbau "Zeiss Visu 200 & S8" ergeben. Änderungen am Gewicht des Mikroskops oder seinem Zubehör können jedoch bei beiden bekannten Lösungen nur durch Änderung der Federkraft der Stützfeder kompensiert werden, d.h., dass alle Gewichtsänderungen zu einer Änderung der Balance um die senkrechte Stützachse bzw. zu geänderten Biegemomenten an dieser führen.

Der Erfindung liegt die Aufgabe zugrunde eine Stativanordnung zu schaffen, die über die senkrechte Stützachse besser im ausbalancierten Gleichgewichtszustand gehalten werden kann, ohne dabei das Gesamtgewicht des Stativaufbaus zu erhöhen.

Gemäss der Erfindung wird dies dadurch erreicht, dass auf der dem Ausleger gegenüber der vertikalen Achse diametral gegenüberliegenden Seite ein Träger zur Aufnahme des Lampengehäuses vorgesehen ist. Da der Träger dem Ausleger gegenüber angeordnet ist, ergeben das Gewicht des Trägers und des Lampengehäuses multipliziert mit dem Abstand ihrer Schwerpunkte von der vertikalen Schwenkachse Gegenmomente, die den am Ausleger entstehenden Drehmomenten entgegenwirken. In der optimale Situation heben sich die Summen dieser Momente gegenseitig auf. Der Stützfuss kann dementsprechend verkleinert und das Gesamtgewicht gleich oder sogar kleiner ausgelegt werden.

Zweckmäßigerweise ist der Träger mit dem Ausleger starr verbunden. Somit entsteht ein zweiarmiger Hebel, entsprechend einem um eine Achse drehbar gelagerten Waagebalken, an dem die einzelnen Kräfte angreifen können. Der Träger und der Ausleger können einteilig ausgebildet oder aus wenigstens zwei Elementen zusammengesetzt sein.

Um sich den unterschiedlichen Gerätekonfigurationen des Mikroskops, beispielsweise durch Anbau von verschiedenen Zubehörteilen anpassen zu können, ist das Lampengehäuse vorteilhaft auf dem Träger verschieb- und feststellbar angeordnet. Diese Verstellbarkeit kann beispielsweise in Stufen durch Lochreihen für die Befestigungsschrauben oder stufenlos durch Längsschlitze erfolgen.

In der einfachsten Bauform ist der Ausleger am Oberteil um die vertikale Drehachse des Ständerfusses schwenkbar. Für eine universelle Verstellbarkeit des Stativs ist jedoch zweckmässig, dass der Ausleger am Oberteil um eine zur vertikalen Drehachse des Ständerfusses parallel verlaufende Achse schwenkbar ist. Somit kann ein Zwischenglied zwischen dem Ständerfuss und dem Ausleger, bzw. Träger bildende Oberteil bei Bedarf rasch weggeschwenkt werden. Das Oberteil kann in diesem Fall ein- oder mehrteilig ausgebildet werden

Das den Transformator, die Glühbirnen und allfällige Regler enthaltende Lampengehäuse ist normalerweise nicht sehr schwer. Für ein besseres Ausbalancieren des Stativs sind daher gemäss einer Weiterbildung der Erfindung am Träger und /oder am Lampengehäuse Befestigungsmittel zur Aufnahme zusätzlicher Ausgleichsgewichte vorgesehen. Solche Befestigungsmittel können beispielsweise Führungsstangen sein, an denen die Ausgleichsgewichte wie bei einer Balkenwaage verschoben und festgelegt werden können.

Anstelle oder zusätzlich zu Ausgleichsgewichten ist es vorteilhaft, zwischen dem Träger und dem Oberteil Federmittel zum Gewichtsausgleich vorzusehen. Solche Federmittel können beispielsweise als einfache Zugfedern oder als Gasdruckfedern ausgebildet werden. Durch sie kann die, am Ausleger gegebenenfalls vorgesehene Stützfeder verkleinert und dadurch das Gewicht des Auslegers selbst reduziert werden, was wiederum dem Balanceeffekt entgegenkommt.

Das Verschieben des Lampengehäuses und/oder der Ausgleichsgewichte kann rein manuell nach Skalen oder nach Gefühl erfolgen. Dieses Vorgehen erfordert jedoch einen Zeitaufwand und eine gewisse Erfahrung oder Geschick. Im Verlauf einer Operation kann es jedoch vorkommen, dass sehr rasch Zubehörteile mit unterschiedlichem Gewicht und Schwerpunktsabstand ausgewechselt werden müssen. Damit dabei keine Verzögerungen oder Komplikationen auftreten, erfolgt das Verschieben des Lampengehäuses und/oder der Ausgleichsgewichte vorteilhaft gemäss einer Weiterbildung der Erfindung über eine automatische Steuerung in Abhängigkeit von der Stellung und/oder dem Gewicht des Mikroskops bzw. seines Zubehörs. Die Steuerung kann beispielsweise über Kraft-, Deformations-, Weg- oder Winkelsensoren geregelt werden. Dabei können auch äußere angreifende Kräfte, beispielsweise das sich Abstützen einer Person am Stativ, kompensiert werden.

Eine Vorrichtung für das automatische Balancieren eines Stativs ist in mehreren Ausführungsbeispielen in der WO 9713997 A1 angegeben. In den Figuren 1 bis 6, 8 und 18 bis 20 und der zugehörigen Beschreibung sind die Automatikfunktionen zum automatischen Balancieren eines Stativs dargestellt und beschrieben.

Die Erfindung soll nachstehend anhand der sie beispielsweise wiedergebenden symbolischen Zeichnungen näher erläutert werden. Es zeigen:
- Fig. 1: Eine erfindungsgemässe Stativanordnung mit um den Ständerfuss drehbar gelagertem Ausleger,
- Fig. 2: eine Teilansicht einer Stativanordnung mit zusätzlichen Gewichten und Federmitteln zum Gewichtsausgleich,
- Fig. 3: eine Stativanordnung mit seitlich versetzter Lagerung des Auslegers.

In den Figuren wurden entsprechende Teile mit gleichen Bezugsziffern versehen. Dabei haben vergleichbare Teile gleiche Bezugszeichen mit unterschiedlichen Indizes.

Der Stativaufbau besteht im wesentlichen aus einem Ständerfuß 1 und einem damit verbundenen Oberteil 2a. Das Oberteil 2a ist gegenüber dem Ständerfuß 1 um eine vertikal verlaufende Achse A drehbar gelagert. Ein Ausleger 3 ist mit dem Oberteil 2a über einen Bolzen 4 beweglich verbunden. Der Bolzen 4 bildet eine im wesentlichen horizontal verlaufene Schwenkachse B. Der Ausleger 3 ist somit um die Achse B, bzw. den Bolzen 4 auf und ab bewegbar. Am freien Ende des Auslegers 3 hängt eine vertikal verlaufende Tragsäule 5. Eine parallel zum Ausleger 3 verlaufende Strebe 6 ist an einem Ende mit dem Oberteil 2a und am anderen Ende mit der Tragsäule 5 gelenkig verbunden. Der Ausleger 3, die Strebe 6, das Oberteil 2a und die Tragsäule 5 bilden somit ein Parallelogramm. Dies bedeutet, dass die Tragsäule 5 unabhängig von der Position des Auslegers 3, wie es durch das Verschwenken des Auslegers in Doppelpfeilrichtung angedeutet ist, stets in vertikaler Lage bleibt. Zum Austarieren des Gewichts an der Tragsäule 5 und des Auslegers 3 ist eine Stützfeder, z.B. eine Gasdruckfeder 7 vorgesehen. Dieser Aufbau mit der Stützfeder entspricht etwa dem Aufbau, wie er aus der EP 0 433 426 A1 und dem "Zeiss Visu 200 & S8" bekannt ist, so dass auf die technischen Einzelheiten nicht näher eingegangen werden muss.

Betont wird, dass die Erfindung nicht auf solche Scherenarmkonstruktionen eingeschränkt ist, sondern vielmehr auch Auslegerkonstruktionen ohne Strebe 6 und Gasdruckfeder 7 umfasst.

Am unteren Ende der Tragsäule 5 befindet sich eine Konsole 8. Diese kann zusätzliche Gelenke oder Verstellmechanismen für ein daran befestigtes Mikroskop 9 aufweisen.

Auf der dem Ausleger 3 gegenüberliegenden Seite der Achse B befindet sich ein Träger 10. Dieser ist vorzugsweise mit dem Ausleger 3 fest verbunden oder einstückig ausgebildet. Der Träger 10 dient der Aufnahme eines Lampengehäuses 11 zum Beleuchten der Operationsstelle. Das Lampengehäuse 11 ist mit dem Träger 10 mittels Schrauben 12 lösbar verbunden. Nach dem Lösen der Schrauben 12 kann das Lampengehäuse 11 somit zum Ausbalancieren des Auslegers 3 in eine andere Stellung längs des Trägers 10 verschoben und dort wieder fixiert werden. Das beispielsweise mittels Glühlampen 18 erzeugte Licht wird über einen flexiblen Lichtleiter 13 dem Sichtfeld des Operateurs zugeführt. Die Schrauben 12 können, wie nicht näher dargestellt, mit Griffen, Knebeln, Rändeln, Flügeln o.dgl. ausgerüstet sein, um deren Handhabung zu vereinfachen. Sie können im Rahmen der Erfindung auch durch beliebige andere Befestigungsmittel ersetzt werden.

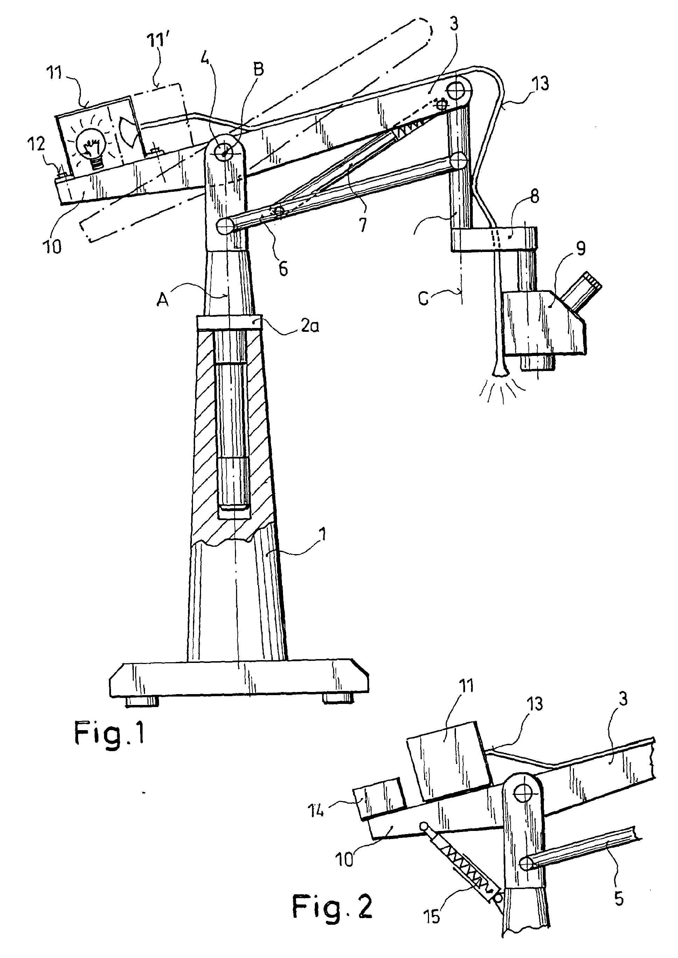

Die aus Fig. 2 ersichtliche Ausführung unterscheidet sich gegenüber der in Fig. 1 dargestellten Ausführung dadurch, dass ausser dem Lampengehäuse 11 und Träger 10 weitere Mittel zum Ausbalancieren des Auslegers 3 vorgesehen sind. Dies kann wahlweise durch zusätzliche am Träger 10 befestigbare Ausgleichsgewichte 14 und/oder beispielsweise als Zugfedern oder Gasdruckfedern ausgebildete Federmittel 15 erfolgen. Die Ausgleichsgewichte 14 können, wie auch das Lampengehäuse 11, verschiebbar ausgebildet sein. Diese Verschiebung erfolgt im Ausführungsbeispiel über einen Motor 20, über den das Ausgleichsgewicht 14 in Doppelpfeilrichtung entlang des Trägers 10 verschoben wird. Der Motor 20 wird über eine Steuereinrichtung 19 mit Strom beaufschlagt. Die Ansteuerung des Motors 20 erfolgt in Abhängigkeit von der Stellung und dem Gewicht des Mikroskops 9 und deren Zubehörteile, wobei die Stellung mit nicht dargestellten Sensoren von der Steuereinrichtung 19 erfasst wird. In einer besonderen Ausgestaltung ist die Steuereinrichtung 19 im Lampengehäuse 11 angeordnet.

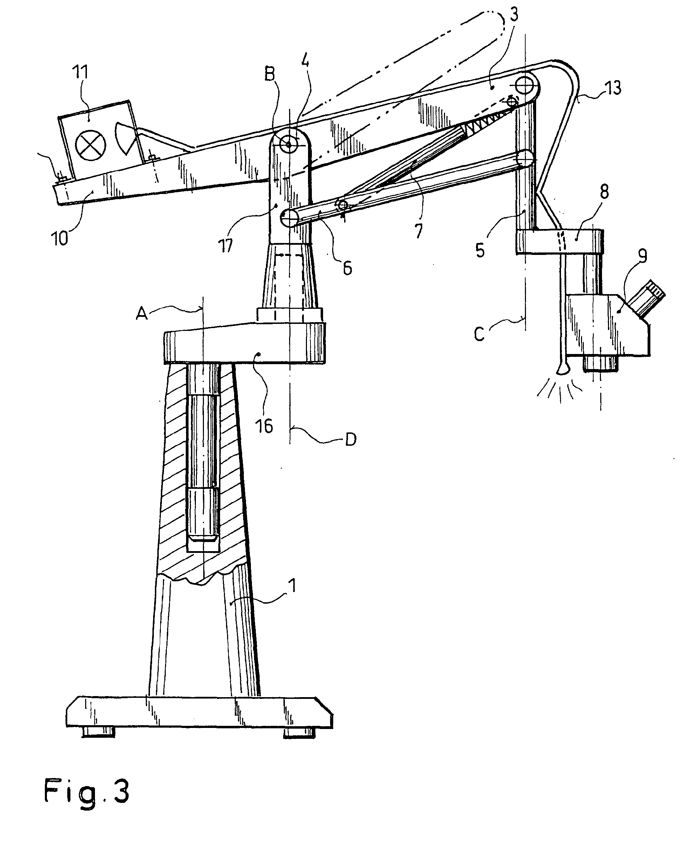

In der in Fig. 3 dargestellten Ausführung ist das Oberteil 2b zweiteilig ausgebildet und besteht aus einer Traverse 16 und einer damit beweglich verbundenen Stütze 17. Diese Anordnung ergibt eine zusätzliche, parallel zur Achse A verlaufende Achse D. Die Stütze 17 und die Traverse 16 können somit nach Art einer Handkurbel um den Ständerfuss 1 herumgedreht werden. Der mit der Stütze 17 verbundene Ausleger 3 sowie auch der Träger 10 können somit in einer parallel zur Zeichnungsebene verlaufenden Lage bewegt werden. Auch bei dieser Lösung können neben dem Lampengehäuse 11 zusätzliche Mittel zum Gewichtsausgleich vorgesehen werden.

In einer bevorzugten Ausgestaltung der Erfindung überragen die Mittel zum Gewichtsausgleich die Achse A, wie dies in der Fig. 1 dargestellt ist. Damit wird in vorteilhafter Weise erreicht, dass der Gewichtsausgleich für jene Stativstellung optimiert ist, in der die weiteste Ausladung des Auslegers 3 von der Achse A weg ragt. In dieser Stativstellung besteht die größte Kippgefahr für das Stativ.

### Bezugszeichenliste

- 1: Ständerfuß
- 2 a, b: Oberteil
- 3: Ausleger
- 4: Bolzen
- 5: Tragsäule
- 6: Strebe
- 7: Gasdruckfeder
- 8: Konsole
- 9: Mikroskop
- 10: Träger
- 11, 11': Lampengehäuse
- 12: Schrauben
- 13: Lichtleiter
- 14: Ausgleichsgewicht
- 15: Federmittel
- 16: Traverse
- 17: Stütze
- 18: Glühlampe
- 19: Steuereinrichtung
- 20: Motor
- A,C,D: erste - dritte vertikale Achse, B horizontale Achse

## Patentansprüche

1. Stativanordnung, insbesondere für medizinische Untersuchungs- und Operationsmikroskope, mit einem Ständerfuss (1), einem gegenüber dem Ständerfuss (1) um eine erste vertikale Achse (A) drehbaren Oberteil (2), an dem ein um eine horizontale Achse (B) schwenkbarer und um die erste vertikale Achse (A) und/oder um eine zweite vertikale Achse (D) verdrehbarer Ausleger (3) zur Aufnahme eines damit verbundenen Mikroskops (9) befestigt ist, wobei am Stativ eine Beleuchtungseinrichtung mit mindestens einem Lampengehäuse (11) und mit wenigstens einem flexiblen Lichtleiter (13) zum Beleuchten eines Objektes vorgesehen sind, **dadurch gekennzeichnet, dass** auf der dem Ausleger (3) gegenüber der ersten und/oder zweiten vertikalen Achse (A, D) diametral gegenüberliegenden Seite ein Träger (10) zur Aufnahme des Lampengehäuses (11) vorgesehen ist.

2. Stativanordnung nach Anspruch 1, **dadurch gekennzeichnet, dass** der Träger (10) mit dem Ausleger (3) starr verbunden oder als einstückiges Bauteil ausgebildet ist.

3. Stativanordnung nach einem der Ansprüche 1 oder 2, **dadurch gekennzeichnet, dass** das Lampengehäuse (11) auf dem Träger (10) verschieb- und feststellbar angeordnet ist.

4. Stativanordnung nach einem der Ansprüche 1 bis 3, **dadurch gekennzeichnet, dass** der Ausleger (3) um eine zur vertikalen Drehachse (A) des Ständerfusses (1) parallel verlaufende Achse (D) am Oberteil (2b) schwenkbar gelagert ist.

5. Stativanordnung nach einem der Ansprüche 1 bis 3, **dadurch gekennzeichnet, dass** der Ausleger (3) zusammen mit dem Oberteil (2a, 2b) um die vertikale Drehachse (A) des Ständerfusses (1) schwenkbar gelagert ist.

6. Stativanordnung nach einem der Ansprüche 1 bis 5, **dadurch gekennzeichnet, dass** am Träger (10) und/oder am Lampengehäuse (11) Befestigungsmittel zur Aufnahme zusätzlicher Ausgleichsgewichte (14) vorgesehen sind.

7. Stativanordnung nach Anspruch 6, **dadurch gekennzeichnet, dass** die Ausgleichsgewichte (14) am Träger (10) verschieb- und feststellbar angeordnet sind.

8. Stativanordnung nach einem der Ansprüche 1 bis 7, **dadurch gekennzeichnet, dass** zwischen dem Träger (10) und dem Oberteil (2a, 2b) Federmittel (15) zum Balanceausgleich vorgesehen sind.

9. Stativanordnung nach Anspruch 3 oder 7, **dadurch gekennzeichnet, dass** das Verschieben des Lampengehäuses (11) und/oder der Ausgleichsgewichte (14) über eine automatische Steuerung (19) mit einem Motor (29) in Abhängigkeit von der Stellung und dem Gewicht des Mikroskops (9) und/oder des Zubehörs erfolgt.

10. Stativanordnung nach einem der vorhergehenden Ansprüche, **dadurch gekennzeichnet, dass** der Träger (10) in wenigstens einer Drehstellung sowohl die zweite vertikale Achse (D) als auch die erste vertikale Achse (A) überragt, so dass der Gewichtsausgleich bei der weitesten Ausladung des Auslegers(3) von der ersten Achse (A) weg optimiert ist.
